# EUROPEAN PATENT APPLICATION

(11) **EP 2 730 228 A1**
(43) Date of publication of application: **14.05.2014**
(21) Application number: 13184288.2
(22) Date of filing: 13.09.2013
(51) Int. Cl.: A61B 8/00

(54) **Ultrasound diagnosis apparatus and method of controlling the same**

(30) Priority: 07.11.2012 KR 20120125388
(71) Applicant: Samsung Medison Co., Ltd., Gangwon-do 250-870 (KR)
(72) Inventor: Jo, Jae Moon, Gyeonggi-do (KR)
(74) Representative: Lorenz, Markus

(57) **Abstract**

An ultrasound diagnosis apparatus includes a control panel through which a command for manipulation of the ultrasound diagnosis apparatus is input, a keyboard configured to input, and a controller configured to determine whether or not an input time of text is reached and to control movement of the keyboard such that the keyboard extends outward.

## Description

### TECHNICAL FIELD

The present inventive concept relates to an ultrasound diagnosis apparatus for generating an internal image of a target object using ultrasound waves.

### BACKGROUND

An ultrasound diagnosis apparatus directs an ultrasound signal from a surface of a target object toward a target part to acquire an image of a cross section of soft tissues or blood flow based on information of a reflected ultrasound signal (an ultrasound echo signal) using a non-invasive method.

The ultrasound diagnosis apparatus is small in size, inexpensive, displays an image in real time, and has high stability due to no radiation exposure as compared with other imaging diagnosis apparatuses such as an X-ray diagnosis apparatus, an X-ray computed tomography (CT) scanner, a magnetic resonance imaging (MRI) apparatus, a nuclear medicine diagnosis apparatus, or the like. Thus, the ultrasound diagnosis apparatus has been extensively used for diagnosis of the heart, abdomen, and urinary system in obstetrics and gynecology.

The ultrasound diagnosis apparatus includes an ultrasound probe to transmit an ultrasound signal to the target object and to receive an ultrasound echo signal reflected from the target object in order to obtain an ultrasound image of the target object.

The ultrasound probe includes a piezoelectric layer for interconverting an electrical signal and an audible signal while piezoelectric materials oscillate, a matching layer for reducing an audible impedance difference between the piezoelectric layer and the target object so as to transmit as much ultrasound waves generated by the piezoelectric layer as possible, a lens for focusing ultrasound waves, which proceed toward the front of the piezoelectric layer, at a specific location, and a sound absorption layer for blocking ultrasound waves from proceeding toward the rear of the piezoelectric layer to prevent image distortion.

In addition, the ultrasound diagnosis apparatus includes a control panel for manipulating an ultrasound image and input information, and a keyboard for inputting text.

### SUMMARY

Therefore, an aspect of the present inventive concept relates to an ultrasound diagnosis apparatus and a method of controlling the same, in which a keyboard is automatically moved outside from a lower portion of a control panel at an input time of text, thereby increasing convenience of text input through the keyboard.

Additional aspects of the inventive concept will be set forth in part in the description which follows and, in part, will be obvious from the description, or may be learned by practice of the inventive concept.

One aspect of the present inventive concept encompasses an ultrasound diagnosis apparatus including a control panel through which a command for manipulation of the ultrasound diagnosis apparatus is input, a keyboard configured to input text, and a controller configured to determine whether or not an input time of text is reached and to control movement of the keyboard such that the keyboard extends outward.

The keyboard may be disposed below the control panel to extend outward.

The controller may be configured to control the movement of the keyboard such that the keyboard extends outward when the controller determines that at least one of an input time of information related to a target object, an input time of text regarding an ultrasound image, and a time for report writing, is reached.

The ultrasound diagnosis apparatus may further include a display to display an ultrasound image and information related to ultrasound diagnosis, wherein the controller may be configured to control the movement of the keyboard such that the keyboard extends outward when at least one of an image for input of information related to a target object, an image for input of text regarding an ultrasound image, and an image for report writing is displayed on the display.

The controller may be configured to control the movement of the keyboard such that the keyboard extends outward when the controller receives at least one of a command for input of information related to a target object, a command for input of text regarding an ultrasound image, and a command for report writing.

The controller may be configured to control movement of the keyboard such that the keyboard moving to extend outward returns to an original position when the controller determines that the text input has been completed.

The controller may be configured to control the movement of the keyboard such that the keyboard moving to extend outward returns to the original position when the controller receives at least one of a command indicating that input of information related to a target object has been completed, a command indicating that input of text regarding an ultrasound image has been completed, and a command indicating that report writing has been completed.

The keyboard may be installed to slide outward from an installed position of the keyboard.

The ultrasound diagnosis apparatus may further include a notification output device to output visual and audible notification during the movement of the keyboard.

Another aspect of the present inventive concept relates to a method of controlling an ultrasound diagnosis apparatus including: determining whether or not an input time of text is reached, moving a keyboard to extend outward when it is determined that the input time of the text is reached, determining whether or not text input has been completed, and returning the keyboard to an original position when it is determined that the text input has been completed.

The determining whether or not the input time of the text is reached may include determining whether at least one of an input time of information related to a target object, an input time of text regarding an ultrasound image, and a time for report writing is reached.

The determining whether or not the input time of the text is reached may include determining whether at least one of an image to input information related to a target object, an image for input of text regarding an ultrasound image, and an image for report writing is displayed on a display.

The determining whether or not the input time of the text is reached may include determining whether at least one of a command for input of information related to a target object, a command for input of text regarding an ultrasound image, and a command for report writing is received.

The moving of the keyboard to extend outward may further include outputting a signal indicating movement of the keyboard through a notification output device while moving the keyboard.

The determining whether or not the text input has been completed may include determining whether at least one of a command indicating that input of information related to a target object has been completed, a command indicating that input of text regarding an ultrasound image has been completed, and a command indicating that report writing has been completed is received.

The returning of the keyboard to the original position may further include outputting a signal indicating movement of the keyboard through a notification output device while moving the keyboard.

According to the present inventive concept, an operator may not have to directly move a keyboard installed below a control panel outward for text input through the keyboard, and thus, the operator may more conveniently input text.

### BRIEF DESCRIPTION OF THE DRAWINGS

The foregoing and other features of the inventive concept will be apparent from more particular description of embodiments of the inventive concept, as illustrated in the accompanying drawings in which like reference characters may refer to the same or similar parts throughout the different views. The drawings are not necessarily to scale, emphasis instead being placed upon illustrating the principles of the embodiments of the inventive concept. In the drawings, the thickness of layers and regions may be exaggerated for clarity.
FIG. 1 is a block diagram of a structure of an ultrasound diagnosis apparatus according to an embodiment of the present inventive concept.
FIG. 2 is a diagram showing movement control of a keyboard of the ultrasound diagnosis apparatus of FIG. 1.
FIG. 3 is a flowchart of a method of controlling an ultrasound diagnosis apparatus according to an embodiment of the present inventive concept.
FIG. 4 is a block diagram of a structure of an ultrasound diagnosis apparatus according to another embodiment of the present inventive concept.
FIG. 5 is a diagram showing movement control of a keyboard of the ultrasound diagnosis apparatus of FIG. 4.
FIG. 6 is a flowchart of a method of controlling an ultrasound diagnosis apparatus according to another embodiment of the present inventive concept.

### DETAILED DESCRIPTION

Examples of the present inventive concept will be described below in more detail with reference to the accompanying drawings. The examples of the present inventive concept may, however, be embodied in different forms and should not be construed as limited to the examples set forth herein. Like reference numerals may refer to like elements throughout the specification.

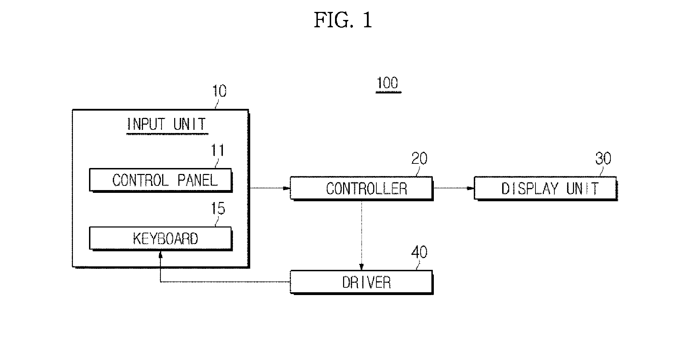

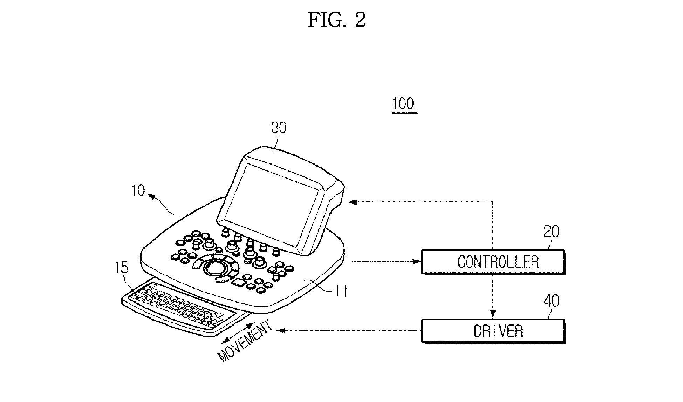

FIG. 1 is a block diagram of a structure of an ultrasound diagnosis apparatus 100 according to an embodiment of the present inventive concept and FIG. 2 is a diagram showing movement control of a keyboard 15 of the ultrasound diagnosis apparatus 100 of FIG. 1.

The ultrasound diagnosis apparatus 100 according to an embodiment of the present inventive concept may include an input device 10 including the keyboard 15 and a control panel 11 through which a command for manipulation of the ultrasound diagnosis apparatus 100 is input, a controller 20 for receiving the command input through the input device 10 to control overall operation of the ultrasound diagnosis apparatus 100, a display 30 for displaying an ultrasound image and various information related to a target object, and a driver 40 for providing driving force for movement of the keyboard 15.

The control panel 11 may include a button, a slide switch, a trackball, and the like for manipulation of the ultrasound diagnosis apparatus 100.

An operator may manipulate the ultrasound image displayed on the display 30 of the ultrasound diagnosis apparatus 100 in various ways, through various input devices included in the control panel 11.

FIG. 2 shows a case in which the keyboard 15 is installed below the control panel 11, which is merely an example. An installed position of the keyboard 15 is not limited thereto. Alternatively, the keyboard 15 may be installed at an arbitrary location of the ultrasound diagnosis apparatus 100. Hereinafter, a case in which the keyboard 15 is installed below the control panel 11 will be exemplified.

Since the keyboard 15 is installed below the control panel 11, the keyboard 15 may not be viewed from outside the ultrasound diagnosis apparatus 100 when the ultrasound diagnosis apparatus 100 is not manipulated, but the keyboard 15 may be exposed outside the ultrasound diagnosis apparatus 100 during text input through the keyboard 15 such that the text may be input through the keyboard 15.

The keyboard 15 may be accommodated in a lower portion of a housing included in the control panel 11 and may be installed at the housing through a sliding device (not separately shown) so as to slide inside and outside the housing. Of course, as the sliding device, various known devices may be employed.

The display 30 may display the ultrasound image and the various information related to the target object. The operator may manipulate an ultrasound probe while checking the ultrasound image displayed on the display 30 and may identify the target object while checking the information related to the target object, displayed on the display 30.

The controller 20 may control the driver 40 to adjust the movement of the keyboard 15. The driver 40 may include a motor to provide power whereby the keyboard 15 may move out of the control panel 11 from a lower portion thereof and move inside the control panel 11.

In general, the operator may manipulate the ultrasound probe with one hand and manipulates the control panel 11 or the keyboard 15 to manipulate an image or to input information with the other hand.

It is inconvenient and cumbersome to pull the keyboard 15 installed below the control panel 11 out of the control panel 11 with one hand for text input, and to return the keyboard 15 to an original position when the text input is completed.

Accordingly, when the controller 20 determines whether or not an input time of the text is reached, if the controller 20 determines that the input time is reached, the controller 20 may control the driver 40 to move the keyboard 15 out of the control panel 11 from the lower portion thereof.

In general, a case in which the operator inputs text through the keyboard 15 may be, for example, a case in which patient relevant information, that is, information such as a patient identification (ID) or personal information is input, a case in which text is input onto an ultrasound image during ultrasound scan, or a case in which a report such as a record regarding an image is written after the ultrasound image is checked.

As described above, when the controller 20 determines that an input time of the information related to the target object, an input time of text regarding the ultrasound image, or a time for report writing, is reached, the controller 20 may control the driver 40 to move the keyboard 15 out of the control panel 11 from the lower portion thereof.

For example, when the controller 20 receives a command for input of the information related to the target object, a command for input of text regarding the ultrasound image, a command for report writing, or the like, the controller 20 may determine that the input time is reached and control the driver 40 to move the keyboard 15 out of the control panel 11.

The command for the text input may be input through the control panel 11. For example, when an icon such as patient information input is displayed on the display 30, if the icon is clicked or touched, it may be considered that the command for input of patient information is input, and thus, the controller 20 may automatically move the keyboard 15 out of the control panel 11.

In addition, when an image requiring input of the information related to the target object, an image requiring input of text regarding the ultrasound image, or a report writing image, is displayed on the display 30 according to the command for the text input, the controller 20 may also determine that an input time of text is reached and control the driver 40 to move the keyboard 15 out of the control panel 11.

Determination of the input time of text is not limited to the aforementioned examples and may be performed in various ways.

When the keyboard 15 is moved out of the control panel 11 from the lower portion thereof and then the text input through the keyboard 15 is completed, the controller 20 may control the driver 40 to return the keyboard 15 to the original position.

That is, when the controller 20 receives a command indicating that the input of the information related to the target object has been completed, a command indicating that input of text regarding the ultrasound image has been completed, or a command indicating that the report writing has been completed, the controller 20 may control the driver 40 to move the keyboard 15 toward the lower portion of the control panel 11 again.

Here, the command indicating that the text input has been completed may correspond to, for example, click, touch, or the like of an icon to store the information related to the target object after the information is input. That is, after the information related to the target object is input, when a storage icon to store the input information is clicked, the controller 20 may determine that the input of the information related to the target object has been completed and cause the keyboard 15 to return to the original position.

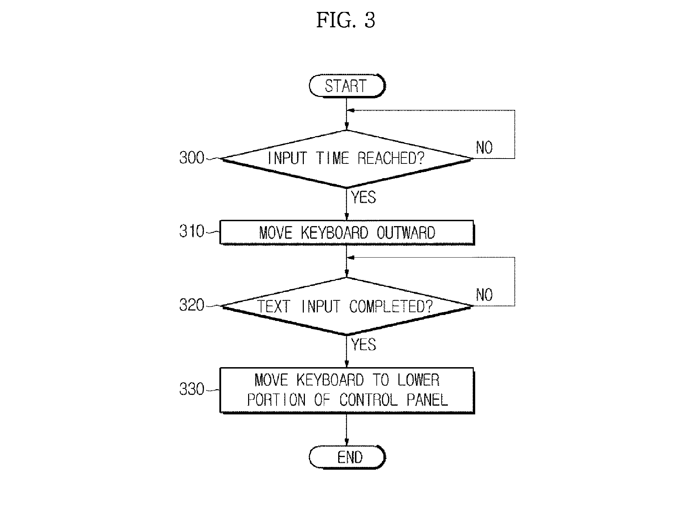

FIG. 3 is a flowchart of a method of controlling the ultrasound diagnosis apparatus 100 according to an embodiment of the present inventive concept.

Referring to FIG. 3, the controller 20 may determine whether or not an input time of text is reached (300). When the controller 20 determines that the input time is reached, the controller 20 may move the keyboard 15 out of the control panel 11 (310).

In general, a case in which the operator inputs text through the keyboard 15 may be, for example, a case in which patient relevant information, that is, information such as a patient identification (ID) or personal information is input, a case in which text is input onto an ultrasound image during ultrasound scan, or a case in which a report such as a record regarding an image is written after the ultrasound image is checked.

As described above, when the controller 20 determines that an input time of the information related to the target object, an input time of text regarding the ultrasound image, or a time for report writing, is reached, the controller 20 may control the driver 40 to move the keyboard 15 out of the control panel 11 from the lower portion thereof.

For example, when the controller 20 receives a command for input of the information related to the target object, a command for input of text regarding the ultrasound image, a command for report writing, or the like, the controller 20 may determine that the input time is reached and control the driver 40 to move the keyboard 15 out of the control panel 11.

The command for the text input may be input through the control panel 11. For example, when an icon such as patient information input is displayed on the display 30, if the icon is clicked or touched, it may be considered that the command for input of patient information is input, and thus, the controller 20 may automatically move the keyboard 15 out of the control panel 11.

In addition, when an image requiring input of the information related to the target object, an image requiring input of text regarding the ultrasound image, or a report writing image is displayed on the display 30 according to the command for the text input, the controller 20 may also determine that an input time of text is reached and control the driver 40 to move the keyboard 15 out of the control panel 11.

Determination of the input time of text is not limited to the aforementioned examples and may be performed in various ways.

When the keyboard 15 is moved out of the control panel 11, the controller 20 may determine whether or not the text input has been completed (320). When the controller 20 determines that the text input has been completed, the controller 20 may move the keyboard 15 to the lower portion of the control panel 11 (330).

When the keyboard 15 is moved out of the control panel 11 from the lower portion thereof and then the text input through the keyboard 15 is completed, the controller 20 may control the driver 40 to return the keyboard 15 to the original position.

That is, when the controller 20 receives, for example, a command indicating that the input of the information related to the target object has been completed, a command indicating that input of text regarding the ultrasound image has been completed, or a command indicating that the report writing has been completed, the controller 20 may control the driver 40 to move the keyboard 15 toward the lower portion of the control panel 11 again.

Here, the command indicating that the text input has been completed may correspond to, for example, click, touch, or the like of an icon to store the information related to the target object after the information is input. That is, after the information related to the target object is input, when a storage icon to store the input information is clicked, the controller 20 may determine that the input of the information related to the target object has been completed and cause the keyboard 15 to return to the original position.

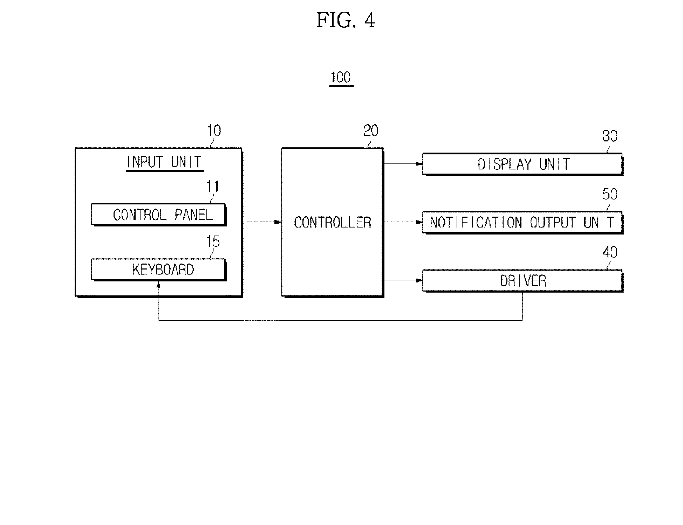

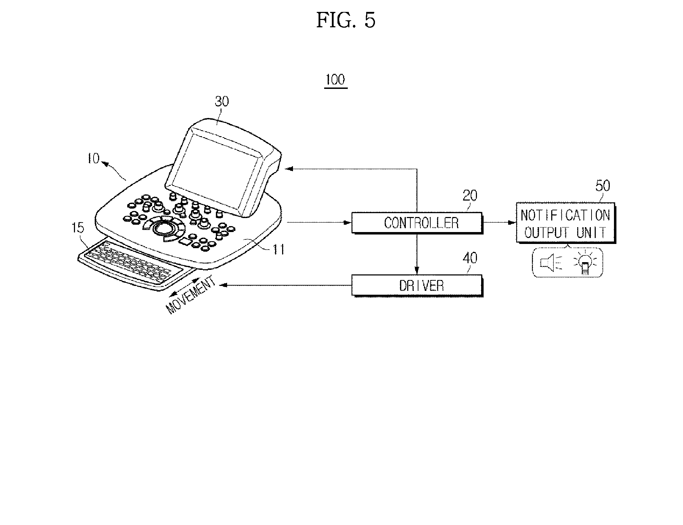

FIG. 4 is a block diagram of a structure of an ultrasound diagnosis apparatus which is designated by reference numeral 100 according to another embodiment of the present inventive concept and FIG. 5 is a diagram showing movement control of the keyboard 15 of the ultrasound diagnosis apparatus 100 of FIG. 4.

The ultrasound diagnosis apparatus 100 according to an embodiment of the present inventive concept includes the input device 10 including the keyboard 15 and control panel 11 through which a command for manipulation of the ultrasound diagnosis apparatus 100 is input, the controller 20 for receiving the command input through the input device 10 to control overall operation of the ultrasound diagnosis apparatus 100, the display 30 for displaying an ultrasound image and various information related to a target object, the driver 40 for providing driving force for movement of the keyboard 15, and a notification output device 50 for externally outputting movement of the keyboard 15 through visual and audible signals when the keyboard 15 is moved.

The structure of the ultrasound diagnosis apparatus 100 of FIGS. 4 and 5 may be the same as the ultrasound diagnosis apparatus 100 of FIGS. 1 and 2, except for the notification output device 50, and thus, a detailed description thereof is omitted herein.

The notification output device 50 may externally output the visual and audible signals indicating the movement of the keyboard 15 when the keyboard 15 is moved out of the control panel 11 from the lower portion thereof or the keyboard 15 moved out of the control panel 11 is moved to the lower portion of the control panel 11 again, so that the operator may recognize the movement of the keyboard 15.

As shown in FIG. 5, the notification output device 50 may indicate the movement of the keyboard 15 by outputting a sound through a speaker or blinking a light. Embodiments of the present inventive concept are not limited to this method of outputting the visual and audible signals. Various other methods may be used as long as the operator is informed of the movement of the keyboard 15.

The notification output device 50 may be disposed on one surface of the ultrasound diagnosis apparatus 100, and in detail, may be disposed on the control panel 11.

When the controller 20 determines that the input time of text is reached, the controller 20 drives the notification output device 50 to notify the operator of the movement of the keyboard 15 while moving the keyboard 15 out of the control panel 11 from the lower portion thereof.

Similarly, when the controller 20 determines that the text input has been completed, the controller 20 may drive the notification output device 50 to notify the operator of the movement of the keyboard 15 while returning the keyboard 15 to the lower portion of the control panel 11.

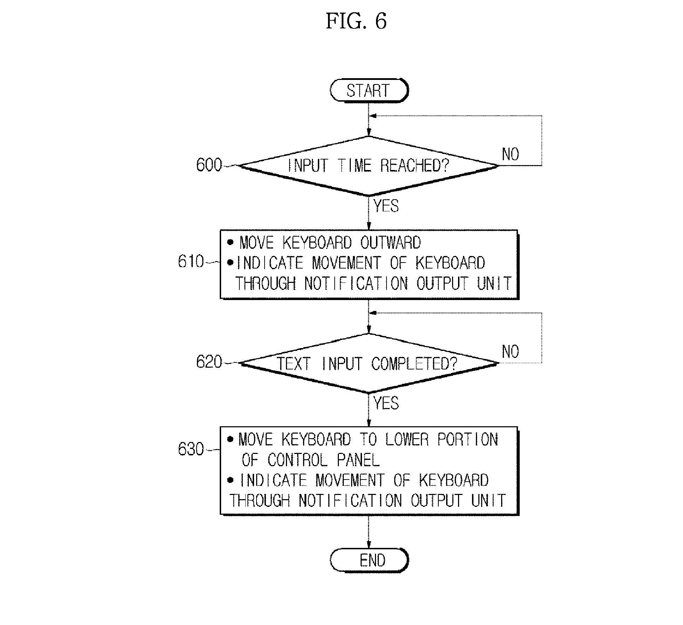

FIG. 6 is a flowchart of a method of controlling the ultrasound diagnosis apparatus 100 according to another embodiment of the present inventive concept.

Referring to FIG. 6, the controller 20 may determine whether or not an input time of text is reached (600). When the controller 20 determines that the input time is reached, the controller 20 may indicate the movement of the keyboard 15 through the notification output device 50 while moving the keyboard 15 out of the control panel 11 (610).

In general, a case in which the operator inputs text through the keyboard 15 may be, for example, a case in which patient relevant information, that is, information such as a patient identification (ID) or personal information is input, a case in which text is input onto an ultrasound image during ultrasound scan, or a case in which a report such as a record regarding an image is written after the ultrasound image is checked.

As described above, when the controller 20 determines that an input time of the information related to the target object, an input time of text regarding the ultrasound image, or a time for report writing, is reached, the controller 20 may control the driver 40 to move the keyboard 15 out of the control panel 11 from the lower portion thereof.

In addition, the controller 20 may drive the notification output device 50 to notify the operator of the movement of the keyboard 15 while moving the keyboard 15 out of the control panel 11 from the lower portion thereof.

For example, when the controller 20 receives, for example, a command for input of the information related to the target object, a command for input of text regarding the ultrasound image, a command for report writing, or the like, the controller 20 may determine that the input time is reached and control the driver 40 to move the keyboard 15 out of the control panel 11.

The command for the text input may be input through the control panel 11. For example, when an icon such as patient information input is displayed on the display 30, if the icon is clicked or touched, it may be considered that the command for input of patient information is input, and thus, the controller 20 may automatically move the keyboard 15 out of the control panel 11.

In addition, when an image requiring input of the information related to the target object, an image requiring input of text regarding the ultrasound image, or a report writing image, is displayed on the display 30 according to the command for the text input, the controller 20 may also determine that an input time of text is reached and control the driver 40 to move the keyboard 15 out of the control panel 11.

Determination of the input time of text is not limited to the aforementioned examples and may be performed in various ways.

When the keyboard 15 is moved out of the control panel 11, the controller 20 may determine whether or not the text input has been completed (620). When the controller 20 determines that the text input has been completed, the controller 20 may move the keyboard 15 to the lower portion of the control panel 11 (630).

When the keyboard 15 is moved out of the control panel 11 from the lower portion thereof and then the text input through the keyboard 15 is completed, the controller 20 may control the driver 40 to return the keyboard 15 to the original position.

That is, when the controller 20 receives, for example, a command indicating that the input of the information related to the target object has been completed, a command indicating that input of text regarding the ultrasound image has been completed, or a command indicating that the report writing has been completed, the controller 20 may control the driver 40 to move the keyboard 15 toward the lower portion of the control panel 11 again.

Here, the command indicating that the text input has been completed may correspond to, for example, click, touch, or the like of an icon to store the information related to the target object after the information is input. That is, after the information related to the target object is input, when a storage icon to store the input information is clicked, the controller 20 may determine that the input of the information related to the target object has been completed and cause the keyboard 15 to return to the original position.

In addition, when the controller 20 determines that the text input has been completed, the controller 20 may drive the notification output device 50 to notify the operator of the movement of the keyboard 15 while returning the keyboard 15 to the lower portion of the control panel 11.

As is apparent from the above description, according to embodiments of the present inventive concept, an operator may not have to directly move a keyboard installed below a control panel out of the control panel for text input through the keyboard, and thus, the operator may more conveniently input text.

Although a few embodiments of the present inventive concept have been shown and described, it would be appreciated by those skilled in the art that changes may be made in these embodiments without departing from the principles and spirit of the inventive concept, the scope of which is defined in the claims and their equivalents.

## Claims

1. An ultrasound diagnosis apparatus, comprising:
a control panel through which a command for manipulation of the ultrasound diagnosis apparatus is input;
a keyboard configured to input; and
a controller configured to determine whether or not an input time is reached and to control movement of the keyboard such that the keyboard extends outward.

2. The ultrasound diagnosis apparatus according to claim 1, wherein the keyboard is disposed below the control panel to extend outward.

3. The ultrasound diagnosis apparatus according to claim 1, wherein the controller is configured to control the movement of the keyboard such that the keyboard extends outward when the controller determines that at least one of an input time of information related to a target object, an input time regarding an ultrasound image, and a time for report writing, is reached.

4. The ultrasound diagnosis apparatus according to claim 1, further comprising a display to display an ultrasound image and information related to ultrasound diagnosis,
wherein the controller is configured to control the movement of the keyboard such that the keyboard extends outward when at least one of an image for input of information related to a target object, an image for input regarding an ultrasound image, and an image for report writing is displayed on the display.

5. The ultrasound diagnosis apparatus according to claim 1, wherein the controller is configured to control the movement of the keyboard such that the keyboard extends outward when the controller receives at least one of a command for input of information related to a target object, a command for input regarding an ultrasound image, and a command for report writing.

6. The ultrasound diagnosis apparatus according to claim 1, wherein the controller is configured to control movement of the keyboard such that the keyboard moving to extend outward returns to an original position when the controller determines that the input has been completed.

7. The ultrasound diagnosis apparatus according to claim 6, wherein the controller is configured to control the movement of the keyboard such that the keyboard moving to extend outward returns to the original position when the controller receives at least one of a command indicating that input of information related to a target object has been completed, a command indicating that input regarding an ultrasound image has been completed, and a command indicating that report writing has been completed.

8. The ultrasound diagnosis apparatus according to claim 1, wherein the keyboard is installed to slide outward from an installed position of the keyboard.

9. The ultrasound diagnosis apparatus according to claim 1, further comprising a notification output device to output visual and audible notification during the movement of the keyboard.

10. A method of controlling an ultrasound diagnosis apparatus, the method comprising:
determining whether or not an input time is reached;
moving a keyboard to extend outward when it is determined that the input time is reached;
determining whether or not input has been completed; and
returning the keyboard to an original position when it is determined that the input has been completed.

11. The method according to claim 10, wherein the determining whether or not the input time is reached comprises determining whether at least one of an input time of information related to a target object, an input time regarding an ultrasound image, and a time for report writing, is reached.

12. The method according to claim 10, wherein the determining whether or not the input time is reached comprises determining whether at least one of an image to input information related to a target object, an image for input regarding an ultrasound image, and an image for report writing, is displayed on a display.

13. The method according to claim 10, wherein the determining whether or not the input time is reached comprises determining whether at least one of a command for input of information related to a target object, a command for input regarding an ultrasound image, and a command for report writing, is received.

14. The method according to claim 10, wherein the moving of the keyboard to extend outward further comprises outputting a signal indicating movement of the keyboard through a notification output device while moving the keyboard.

15. The method according to claim 10, wherein the determining whether or not the input has been completed comprises determining whether at least one of a command indicating that input of information related to a target object has been completed, a command indicating that input regarding an ultrasound image has been completed, and a command indicating that report writing has been completed, is received.

16. The method according to claim 10, wherein the returning of the keyboard to the original position further comprises outputting a signal indicating movement of the keyboard through a notification output device while moving the keyboard.
